# EUROPÄISCHE PATENTSCHRIFT

(11) **EP 0 550 004 B1**
(45) Veröffentlichungstag und Bekanntmachung des Hinweises auf die Patenterteilung: **30.08.1995**
(21) Anmeldenummer: 92121858.2
(22) Anmeldetag: 23.12.1992
(51) Int. Cl.: A61M 5/14, A61J 1/14

(54) **Lichtschutzvorrichtung für den lichtempfindlichen Inhalt einer Infusionsflasche**
Light protecting device for the light-sensitive content of an infusion bottle
Dispositif de protection contre la lumière pour le contenu d'une bouteille de perfusion sensible à la lumière

(30) Priorität: 31.12.1991 DE 4143231
(43) Veröffentlichungstag der Anmeldung: 07.07.1993
(73) Patentinhaber: WÖRWAG PHARMA GmbH, D-70499 Stuttgart (DE)
(72) Erfinder: Haller, Claus-Peter, Dr., W-7016 Gerlingen (DE); Wörwag, Fritz, Dr., W-7000 Stuttgart 1 (DE)
(74) Vertreter: Weller, Wolfgang, Dr.rer.nat.

(56) Entgegenhaltungen:
- EP-A- 0 038 312
- DE-A- 3 702 157
- US-A- 4 048 997

## Beschreibung

Die Erfindung betrifft eine Lichtschutzvorrichtung für den lichtempfindlichen Inhalt einer Infusionsflasche.

Infusionsflaschen werden üblicherweise aus farblosem durchsichtigen Glas oder durchsichtigen Kunststoff hergestellt. Im praktischen Einsatz werden Infusionsflaschen an einer Aufhängevorrichtung aufgehängt, und zwar derart, daß der Boden der Infusionsflasche, in Schwerkraftrichtung gesehen, oberhalb des mit einem durchstechbaren Verschlußstopfen versehenen Auslaufendes zum Liegen kommt. Damit die Infusionsflasche an die Aufhängevorrichtung angehängt werden kann, ist im Bereich des Bodens der Infusionsflasche eine Lasche vorgesehen, mittels derer die Infusionsflasche an ein hakenförmiges Teil der Aufhängevorrichtung eingehängt werden kann. Unter Infusionsflasche im Sinne der vorliegenden Anmeldung sind auch beutelartige Gefäße zu verstehen, die einerseits mit einer solchen Lasche zum Anhängen an einer Aufhängevorrichtung und andererseits mit einem Verschluß versehen sind, durch den eine Kanüle hindurchstechbar ist, um die Infusionslösung aus dem Infusionsgefäß ausfließen lassen zu können. Bei Infusionsflaschen ist dies gewöhnlicherweise ein weichelastischer Kunststoffstopfen, durch den Kanülen hindurchstechbar sind.

Aufgrund der Ausbildung der Infusionsflaschen aus durchsichtigem Material (Glas, Kunststoff) kann im praktischen Einsatz eine visuelle Kontrolle des jeweiligen Füllzustandes einfach durchgeführt werden.

Es treten nun Situationen auf, in denen den Infusionslösungen, meist wäßrige Kochsalzlösungen, zusätzlich Medikamente hinzugefügt werden müssen, die lichtempfindlich sind. Werden solche lichtempfindlichen Substanzen bereits herstellerseits zugegeben, so ist es möglich, die Infusionsflasche selbst bereits aus einem lichtundurchlässigen Material herzustellen oder mit einem lichtundurchlässigen Material zu beschichten, was jedoch sehr kostenaufwendig ist.

Es ist daher Aufgabe der vorliegenden Erfindung, eine Lichtschutzvorrichtung für den lichtempfindlichen Inhalt von Infusionsflaschen zu schaffen, die einfach aufgebaut und kostengünstig herstellbar ist, die im Bedarfsfalle einen Lichtschutz für den lichtempfindlichen Inhalt einer an sich lichtdurchlässigen Infusionsflasche schafft, und die die Handhabung der Infusionsflasche im praktischen Einsatz nicht hindert.

Erfindungsgemäß wird die Aufgabe dadurch gelöst, daß eine lichtundurchlässige Schutzfolie vorgesehen ist, die im Bedarfsfalle nachträglich auf die Infusionsflasche aufbringbar ist und die derart ausgebildet ist, daß im Bodenbereich der Infusionsflasche Aufhängevorrichtungen für die Infusionsflasche durch die Schutzfolie hindurchbringbar sind, und daß im Auslaufbereich der Infusionsflasche eine Öffnung in der Schutzfolie vorgesehen ist.

Eine lichtundurchlässige Schutzfolie ist sehr kostengünstig als Kunststoffolie herstellbar, der beispielsweise Ruß zum Lichtundurchlässigmachen beigemischt ist. Die lichtundurchlässige Schutzfolie selbst muß keine mechanischen Belastungen ertragen, so daß eine solche Folie sehr dünn ausgebildet sein kann. Es ist daher einfach möglich, diese Schutzfolie im Bodenbereich der Infusionsflasche zu durchstoßen, um beispielsweise einen Haken einer Aufhängevorrichtung durch die den Bodenbereich abdeckende Schutzfolie hindurchzubringen und an die am Bodenbereich der Infusionsflasche vorgesehene Lasche anzuhängen. Durch die Elastizität des Folienmaterials kann sich die dadurch gebildete Öffnung nach Durchschieben des hakenförmigen Aufhängeteils wieder so weit schließen oder an dieses Teil anlegen, daß im Bereich dieser Öffnung nach wie vor ein ausreichender Lichtschutz gewährleistet ist. Das Vorsehen einer Öffnung in der Schutzfolie im Auslaufbereich der Infusionsflasche, der ja in Schwerkraftrichtung gesehen am unteren Ende der Infusionsflasche vorhanden ist, ermöglicht die notwendigen Manipulationen an der Infusionsflasche. Diese Manipulationen beinhalten beispielsweise das Einstechen der Ausflußkanüle und auch das Einbringen der lichtempfindlichen Substanz in die Infusionsflasche selbst. Zur einfacheren Handhabung ist es selbstverständlich auch möglich, nach Anlegen der lichtundurchlässigen Schutzfolie die Infusionsflasche so zu halten, daß das Auslaufende in Schwerkraftrichtung nach oben zeigt, und in dieser Stellung die lichtempfindliche Substanz einzubringen, und anschließend die mit der lichtundurchlässigen Folie geschützte Infusionsflasche mit dem Auslaufbereich nach unten weisend an die Aufhängevorrichtung anzuhängen. Die Öffnung kann dabei so gewählt werden, daß gerade der Verschlußstopfen der Infusionsflasche frei zugänglich ist, der selbst normalerweise lichtundurchlässig ist, so daß die notwendigen Manipulationen auch nach Anlegen der lichtundurchlässigen Schutzfolie ohne Behinderung möglich sind.

Derartige Schutzfolien sind einfach und kostengünstig herstellbar, sie sind außerdem sehr raumsparend staubar und können Standard-Infusionsflaschen beigelegt werden, so daß sie im Bedarfsfalle, also falls in die Infusionsflasche eine lichtempfindliche Substanz eingebracht werden soll, nachträglich angebracht werden können.

Somit wird die Aufgabe vollkommen gelöst.

In einer vorteilhaften Ausgestaltung der Erfindung ist die Schutzfolie schlauchartig ausgebildet und ist auf die Infusionsflasche aufschiebbar.

Diese Maßnahme hat den Vorteil, daß durch die schlauchartige Ausbildung die Schutzfolie sehr einfach auf die Infusionsflasche aufbringbar ist, wobei das Aufschieben insbesondere noch dadurch erleichtert ist, daß sich die Infusionsflaschen üblicherweise im Auslaufbereich verjüngen, so daß die schlauchartige Schutzfolie einfach an diesem verjüngten Bereich angesetzt und übergeschoben werden kann.

In einer weiteren Ausgestaltung der Erfindung ist die Schutzfolie als einseitig geschlossener Beutel ausgebildet, der über die Infusionsflasche schiebbar ist.

Diese Maßnahme hat den Vorteil, daß der geschlossene Boden des Beutels als Anschlag dient, der Boden somit ein weiteres Aufschieben hindert, falls er mit dem entsprechenden Teil der Infusionsflasche in Berührung tritt, entweder Boden oder Auslaufende, je nachdem, von welcher Seite her der Beutel über die Infusionsflasche geschoben wurde.

In einer weiteren Ausgestaltung der Erfindung ist der Beutel vom Boden der Infusionsflasche her auf diese aufschiebbar, so daß bei aufgeschobenem Beutel der Boden der Infusionsflasche in der Nähe des geschlossenen Bodens des Beutels zum Liegen kommt.

Diese Maßnahme hat den Vorteil, daß die Infusionsflasche bodenseitig und umfänglich vollkommen von dem aus lichtundurchlässigem Material hergestellten Beutel umschlossen ist, so daß durch den einfach aufschiebbaren Beutel ein Rundumlichtschutz geschaffen wird, der zugleich in der nach unten gerichteten Öffnung die Manipulationen am Auslaufende der Infusionsflasche ermöglicht.

In einer weiteren Ausgestaltung der Erfindung ist im Boden des Beutels eine Öffnung vorgesehen, durch die die Aufhängevorrichtung reicht.

Diese Maßnahme hat den Vorteil, daß das Anbringen der Aufhängevorrichtung an die Lasche am Boden der Infusionsflasche einfach zu bewerkstelligen ist, ohne daß dabei das Schutzfolienmaterial durchstoßen werden muß, was eine gewisse Aufmerksamkeit notwendig macht, damit dies auch an der richtigen Stelle erfolgt.

In einer weiteren Ausgestaltung der Erfindung ist die Öffnung im Boden des Beutels als Schlitz ausgebildet.

Diese Maßnahme hat den Vorteil, daß diese Öffnung bei der Herstellung sehr einfach zu bewerkstelligen ist, wobei der Schlitz durch Spreizen des Kunststoffmaterials im Bereich des Schlitzes eine relativ große Öffnung zum Durchschieben eines hakenförmigen Teiles der Aufhängevorrichtung schafft, die anschließend durch entsprechendes Straffziehen des Beutels wieder sehr eng geschlossen werden kann, so daß durch die notwendige Schlitzöffnung kein oder nur eine unbedenkliche Menge an Licht hindurch treten kann.

In einer weiteren Ausgestaltung der Erfindung ist im Boden des Beutels eine Perforation eingebracht, die zur Schaffung der Öffnung für den Durchtritt der Aufhängevorrichtung aufreißbar ist.

Diese Maßnahme hat nun den beachtlichen Vorteil, daß je nach Ausgestaltung des durch den Boden des Beutels durchzubringenden Teiles der Aufhängevorrichtung die Perforation entsprechend weit aufgerissen werden kann und die entsprechend große Öffnung zum Durchtreten des Teils geschaffen werden kann. Insbesondere dann, wenn die Perforation längs einer Linie parallel zu den seitlichen Längskanten verläuft, entsteht wieder eine Schlitzöffnung, die nach Durchführen der Aufhängevorrichtung wieder eng an dieser anliegend gestrafft werden kann, so daß ein Lichtdurchtritt verhindert werden kann.

In einer weiteren Ausgestaltung der Erfindung ist die Schutzfolie mit Einschnürmitteln versehen, die ein Einschnüren der Schutzfolie nach Aufbringen auf die Infusionsflasche ermöglichen.

Diese Maßnahme hat nun den beträchtlichen Vorteil, daß die Öffnungen - sei es im Auslaufbereich der Infusionsflasche und/oder im Bodenbereich der Infusionsflasche - beim Anlegen der Schutzfolie zunächst sehr groß sein können, so daß beispielsweise die gesamte Bodenfläche der Infusionsflasche frei zugänglich ist, das heißt, daß die Infusionsflasche an die Aufhängevorrichtung angehängt werden kann. Durch Straffen der Einschnürmittel kann dann ein eng anliegender, somit einen Lichtdurchtritt verhindernder Sitz der Schutzfolie sowohl im Bereich des Bodens als auch im Bereich des Auslaufendes der Infusionsflasche bewerkstelligt werden. So kann es beispielsweise möglich sein, die Schutzfolie als beidseitig offenen Schlauch herzustellen, an dessen Enden jeweils Einschnürmittel vorhanden sind. Es ist selbstverständlich auch möglich, die Schutzfolie als einseitig geschlossenen Beutel herzustellen, bei dem im Bereich seiner Öffnung die Einschnürmittel vorgesehen sind.

In einer weiteren vorteilhaften Ausgestaltung der Erfindung weisen die Einschnürmittel zumindest einen umfänglich um die Schutzfolie angeordneten Faden auf.

Diese Maßnahme hat den Vorteil, daß die Einschnürmittel sehr einfach und kostengünstig herstellbar sind und die Handhabung der Schutzfolie beim Anbringen sehr erleichtert ist. Es ist außerdem auch möglich, die Einschnürmittel in Form eines Fadens zur Überprüfung des Füllstandes des Inhaltes der Infusionsflasche kurz zu öffnen und die Schutzfolie zu spreizen und nach der visuellen Kontrolle durch Straffen des Fadens wieder zu schließen.

In einer weiteren vorteilhaften Ausgestaltung der Erfindung ist ein laschenartiger Bereich der Folie von dieser abhebbar und wieder rücklegbar, wodurch bei abgehobenem laschenartigen Bereich eine Öffnung in der Schutzfolie kurzzeitig geschaffen wird, durch die hindurch der Inhalt der durch die Schutzfolie ummantelten Infusionsflasche visuell kontrollierbar ist.

In einer weiteren Ausgestaltung der Erfindung ist die Schutzfolie mit einem Fortsatz versehen, der eine von der Infusionsflasche abführende Leitung umhüllt, so daß eine von der Infusionsflasche durch die Leitung abgeführte Flüssigkeit vor Lichteinwirkung geschützt ist.

Diese Maßnahme hat den Vorteil, daß bei extrem lichtempfindlichen Substanzen auch die von der Infusionsflasche abführende Leitung, die meist aus einem durchsichtigen Kunststoffschlauch besteht, nachträglich mit der lichtundurchlässigen Schutzfolie umhüllt werden kann. Dadurch sind dann die lichtempfindlichen Substanzen im Laufweg von der Infusionsflasche bis zur Infusionsstelle am Körper des Patienten ebenfalls vor Lichteinwirkung geschützt.

In einer weiteren Ausgestaltung der Erfindung ist der Fortsatz als Schlauch ausgebildet.

Diese Maßnahme hat den Vorteil, daß der Fortsatz ohne raumergreifende Maßnahmen die von der Infusionsflasche abführende Leitung, die ebenfalls eine Schlauchform aufweist, umgibt.

In einer weiteren Ausgestaltung der Erfindung ist der Fortsatz als Streifen ausgebildet, der durch Umlegen um die Leitung zu einem schlauchartigen Gebilde formbar ist.

Diese Maßnahme hat den Vorteil, daß das Anlegen des Fortsatzes um die Leitung einfach zu bewerkstelligen ist. Dazu wird der Streifen an die Leitung gelegt und anschließend um diese derart herumgebogen, daß dabei ein schlauchartiges Gebilde geformt wird. Durch Ausbildung des Materials, aus dem der Streifen hergestellt ist, als mit sich selbst haftendes Adhäsionsmaterial reicht dieses Formen durch Umlegen um die Leitung herum bereits aus, einen lichtdichten Abschluß um die Leitung zu bilden.

In einer weiteren Ausgestaltung der Erfindung ist der Streifen längs einer Längskante, die längs zu der schützenden Leitung verläuft, mit einem Haftmittel versehen.

Diese Maßnahme hat nunmehr den Vorteil, daß der Streifen mittels des Haftmittels durch Umlegen um die Leitung zu einem schlauchartigen Gebilde geformt werden kann, das längs der Längskante fest haftend verschlossen ist.

In einer weiteren Ausgestaltung der Erfindung ist der Fortsatz als getrenntes Teil vorgesehen, das nachträglich an die Schutzfolie haftend anbringbar ist.

Diese Maßnahme hat zum einen den Vorteil, daß der Fortsatz, insbesondere, wenn er als Streifen geformt ist, einfach hergestellt und auch raumsparend zusammengelegt zusammen mit der Schutzfolie gelagert werden kann. Zum anderen besteht der erhebliche Vorteil in der Handhabung dadurch, daß das Anlegen der Schutzfolie um die Infusionsflasche und das Anlegen des Fortsatzes um die von der Infusionsflasche abführende Leitung mit zwei zunächst getrennten Teilen bewerkstelligt werden kann, und erst anschließend wird der bereits in lichtundurchlässiger Art und Weise um die Leitung gelegte Fortsatz mit der Schutzfolie, die die Infusionsflasche umhüllt, verbunden, beispielsweise durch eine Klebeverbindung oder dergleichen.

Es versteht sich, daß die vorstehend genannten und nachstehend noch zu erläuternden Merkmale nicht nur in der angegebenen Kombination sondern auch in anderen Kombinationen oder in Alleinstellung einsetzbar sind, ohne den Rahmen der vorliegenden Erfindung zu verlassen. Die Erfindung wird nachstehend an Hand einiger ausgewählter Ausführungsbeispiele in Zusammenhang mit den beiliegenden Zeichnungen näher beschrieben und erläutert.

Es zeigen:
- Fig. 1: eine Seitenansicht eines ersten Ausführungsbeispiels einer erfindungsgemäßen Lichtschutzvorrichtung,
- Fig. 2: eine der Fig. 1 entsprechende Seitenansicht eines weiteren Ausführungsbeispiels einer erfindungsgemäßen Schutzvorrichtung,
- Fig. 3: ein weiteres Ausführungsbeispiel einer erfindungsgemäßen Lichtschutzvorrichtung, die an eine an einer Aufhängevorrichtung aufgehängten Infusionsflasche aufgebracht ist,
- Fig. 4a: ein weiteres Ausführungsbeispiel einer erfindungsgemäßen Lichtschutzvorrichtung, wobei ein separates streifenförmiges Teil dargestellt ist, das zum Umlegen um eine von einer Infusionsflasche abführende Leitung vorgesehen ist,
- Fig. 4b: den in Fig. 4a gezeigten Streifen zu einem schlauchartigen Gebilde geformt, und
- Fig. 5: abschnittsweise eine der Fig. 3 vergleichbare Darstellung einer Lichtschutzvorrichtung, die mit dem in Fig. 4b dargestellten schlauchartigen Gebilde zum Schutz der Infusionsleitung versehen ist.

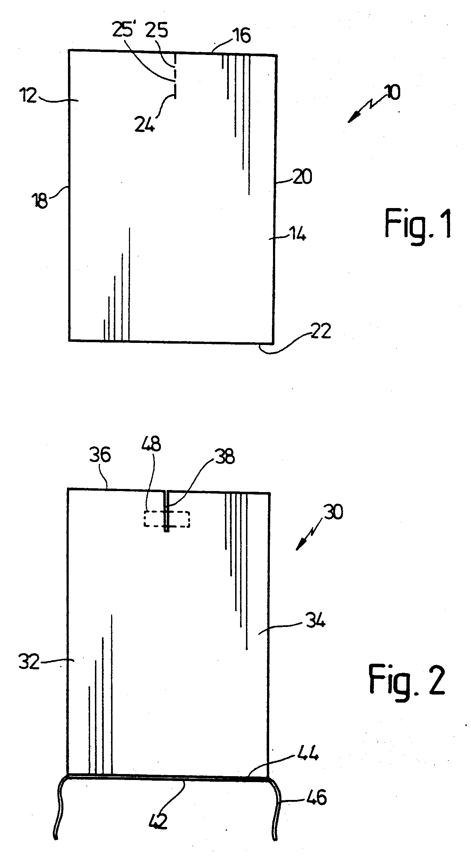

Ein in Fig. 1 dargestelltes erstes Ausführungsbeispiel einer erfindungsgemäßen Lichtschutzvorrichtung 10 besteht aus einer Schutzfolie 12 in Form eines Beutels 14.

Die Schutzfolie 12 besteht aus einem Kunststoffmaterial, wie Polyethylen, Polypropylen, Polycarbonat oder dergleichen, dem zur Lichtundurchlässigmachung des Materials ein Farbstoff, insbesondere feinst verteilter Ruß, hinzugefügt ist.

Der Beutel 14 weist einen geschlossenen Boden 16 auf, der durch seitliches Umlegen eines bandförmigen Kunststoffmaterials entstanden ist, wie das allgemein auf dem Gebiet der Beutelherstellung üblich ist.

Der Beutel 14 ist über Längsschweißnähte 18, 20 seitlich verschlossen. An dem dem Boden 16 gegenüberliegenden Ende ist eine Öffnung 22 vorgesehen, die über die gesamte Breite des Beutels 14 reicht.

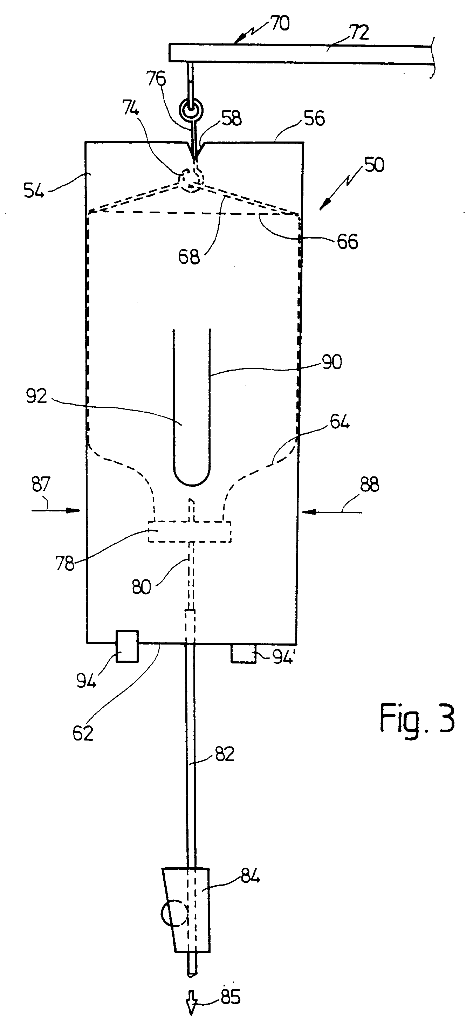

Im Bereich des Bodens 16 ist eine Perforation 24 vorgesehen, die etwa mittig zwischen den beiden Längsschweißnähten 18, 20 und parallel zu diesen verläuft. Die Perforation reicht durch beide Beutelseiten hindurch, so daß durch Aufreißen der Perforation eine Schlitzöffnung hergestellt werden kann. Dazu kann der Beutel 14 im Bereich des Bodens 16 links und rechts der Perforation 24 ergriffen werden und seitlich nach außen bewegt werden, wobei gerade so viel Materialstege 25 und/oder 25' aufgerissen werden, um eine ausreichend große Öffnung im Boden 16 zu bewerkstelligen, damit Teile einer Aufhängevorrichtung durchgeführt werden können, wie dies noch näher an Hand von Fig. 3 beschrieben wird.

Bei einem in Fig. 2 dargestellten weiteren Ausführungsbeispiel einer erfindungsgemäßen Lichtschutzvorrichtung 30 ist die Schutzfolie 32 ebenfalls aus elastischem lichtundurchlässigem Kunststoffmaterial hergestellt, wie das zuvor in Zusammenhang mit Fig. 1 beschrieben wurde.

Auch die Schutzfolie 32 ist als Beutel 34 ausgebildet, dessen Boden 36 geschlossen ist, und der am gegenüberliegenden Ende mit einer Öffnung 42 versehen ist, die über die gesamte Breite des Beutels 34 reicht.

Im Boden 36 ist ein Schlitz 38 vorgesehen, der durch die beiden übereinanderliegenden Beutelseiten hindurch reicht.

Der Schlitz 38 kann beim Herstellen des Beutels 34 einfach dadurch bewerkstelligt werden, daß mittels einer Schneidekante der Schlitz 38 eingestanzt wird.

Ein die Öffnung 42 umgebender Rand oder Bund 44 dient dazu, um einen Faden 46 aufzunehmen, der in den Seitenbereichen des Beutels 34 in hier nicht näher dargestellten Öffnungen aus dem Rand herausragt.

Mittels des Fadens 46 kann der Rand 44 derart gerafft werden, daß die Öffnung 42 auf ein beliebiges Maß verkleinert werden kann.

Es ist ferner vorgesehen, einen Streifen 48 im Bereich des Schlitzes 38 vorzusehen, der dazu dient, den Schlitz 38, nachdem durch diesen eine Aufhängevorrichtung, wie sie in Zusammenhang mit Fig. 3 beschrieben wird, durchgeschoben worden ist, lichtundurchlässig zu verschließen. Der Streifen 48 kann an einem Ende irreversibel und am anderen Ende reversibel haftend am Beutel 34 angebracht sein, oder auch völlig abziehbar angebracht sein.

Es ist bei dem in Fig. 2 dargestellten Ausführungsbeispiel selbstverständlich auch möglich, anstelle des durchgehenden Schlitzes 38 eine Perforation vorzusehen, wie sie im in Fig. 1 dargestellten Ausführungsbeispiel gezeigt ist.

In weiteren, hier nicht dargestellten Ausführungsbeispielen ist vorgesehen, den Beutel als beidseitig offenen Schlauch auszubilden und an beiden offenen Enden entsprechende Ränder oder Bünde 44 vorzusehen, die zur Aufnahme von Fäden 46 dienen. Dadurch ist es dann möglich, den an beiden Seiten offenen Beutel mittels der Fäden 46 auf ein entsprechendes Öffnungsmaß zu raffen.

In Fig. 3 ist ein weiteres Ausführungsbeispiel einer erfindungsgemäßen Lichtschutzvorrichtung 50 dargestellt, die ebenfalls, wie in Zusammenhang mit Fig. 1 und 2 beschrieben, aus einem Beutel 54 aus lichtundurchlässigem Kunststoffmaterial besteht, in dessen geschlossenem Boden 56 eine Schlitzöffnung 58 vorgesehen ist. An dem dem geschlossenen Boden 56 gegenüberliegenden Ende ist der Beutel 54 mit einer Öffnung 62 versehen.

Der Beutel 54 ist über eine Infusionsflasche 64 geschoben, und zwar von seiten deren Boden 66 her.

Im Bereich des Bodens 66 ist die Infusionsflasche 68 mit einer Schlaufe 68 versehen, über die die Infusionsflasche 64 an einer Aufhängevorrichtung 70 angehängt werden kann.

Die Aufhängevorrichtung 70 ist als Galgen 72 ausgebildet, der an seinem vorderen frei ausragenden Ende einen Haken 74 aufweist. Der Haken 74 dient dazu, in die Schlaufe 68 am Boden 66 der Infusionsflasche 64 eingehängt zu werden.

Ein Stiel 76 des Hakens 74 reicht durch die Schlitzöffnung 58 im Boden 56 des Beutels 54 hindurch.

Wie bereits zuvor in Zusammenhang mit Fig. 1 und 2 beschrieben, kann die Schlitzöffnung 58 durch seitliches Spreizen des Bodens 56 des Beutels 54 zu einer entsprechenden Öffnung aufgeweitet werden, die ausreichend ist, daß, nachdem der Beutel 54 über die Infusionsflasche 64 geschoben wurde, der Haken 74 durch die Schlitzöffnung 58 hindurchgebracht und in die Schlaufe 68 eingehängt werden kann. Anschließend schließt sich die Schlitzöffnung 58 wieder zu einem Schlitz, wodurch dann der Boden 56 eng an dem Haken 74 bzw. dessen Stiel 76 anliegt, so daß ein lichtundurchlässiger Abschluß zwischen Schlitzöffnung 58 und Haken 74 bewerkstelligt wird. Es ist auch möglich, einen absolut lichtundurchlässigen Abschluß durch Anlegen eines Klebe- oder Haftstreifens 48, wie er in Zusammenhang mit Fig. 2 beschrieben wurde, zu bewerkstelligen.

In dem in Fig. 3 dargestellten Ausführungsbeispiel ist der Beutel 54 mit einer solchen Länge versehen, daß er einen Verschluß 78 der Infusionsflasche 64 überragt. Durch die Öffnung 62 am unteren Ende des Beutels 54, die über die gesamte Breite des Beutels 54 reicht, ist es möglich, daß beispielsweise eine Kanüle 80 durch den Verschluß 78 der Infusionsflasche 64 hindurchgestochen werden kann, so daß dann über einen Schlauch 82 der Inhalt der Infusionsflasche 64 abfließen kann. Die dazu üblicherweise vorgesehenen Reguliervorrichtungen 84 werden durch den aufgeschobenen Beutel 54 nicht gehindert.

Es ist nun möglich, das abhängende überstehende Ende des Beutels 54 nach Anlegen der notwendigen Auslaufvorrichtungen so zu belassen, wie dies in Fig. 3 dargestellt ist, da das verlängerte, über den Verschluß 78 reichende Ende des Beutels 54 bereits dafür sorgen kann, daß ein ausreichender Lichtschutz des Inhalts der Infusionsflasche 64 bewerkstelligt ist.

Es ist selbstverständlich auch möglich, den überhängenden Beutel 54 im Bereich des Verschlusses 78 einzuschnüren, beispielsweise durch äußeres Anlegen eines Gummiringes oder eines Fadens, wie dies in Fig. 3 durch die Pfeile 87 bzw. 88 angedeutet ist.

Es ist selbstverständlich auch möglich, das in Fig. 3 dargestellte Ausführungsbeispiel mit Einschnürmitteln in Form eines Fadens 46 zu versehen, wie dies in Zusammenhang mit Fig. 2 beschrieben wurde.

Bei dem in Fig. 3 dargestellten Beutel 54 ist eine U-förmige Stanzlinie 90 eingebracht, bei der die Schenkel des "U" parallel zu den Seitenlängslinien des Beutels 54 verlaufen, wobei das "U" zum Boden 56 des Beutels hin öffnet.

Dadurch wird eine Lasche 92 gebildet, die am unteren Ende ergriffen werden kann und angehoben werden kann, um dadurch visuell den Inhalt der durch den lichtundurchlässigen Beutel 54 ummantelten Infusionsflasche 64 kontrollieren zu können.

Im Bereich der Öffnung 62 können auch Haft- oder Klebestreifen oder -laschen 94, 94' vorgesehen sein, die, nach einem Raffen des Beutels 54 um den Schlauch 82, dazu dienen, das geraffte Ende verschlossen zu halten, so daß durch die Öffnung 62 kein Licht in den Innenraum des Beutels 54 eindringen kann. Auch hier können die Streifen oder Laschen 94, 94' integriert, einseitig oder beidseitig reversibel oder irreversibel haftend am Beutel 54 angebracht sein.

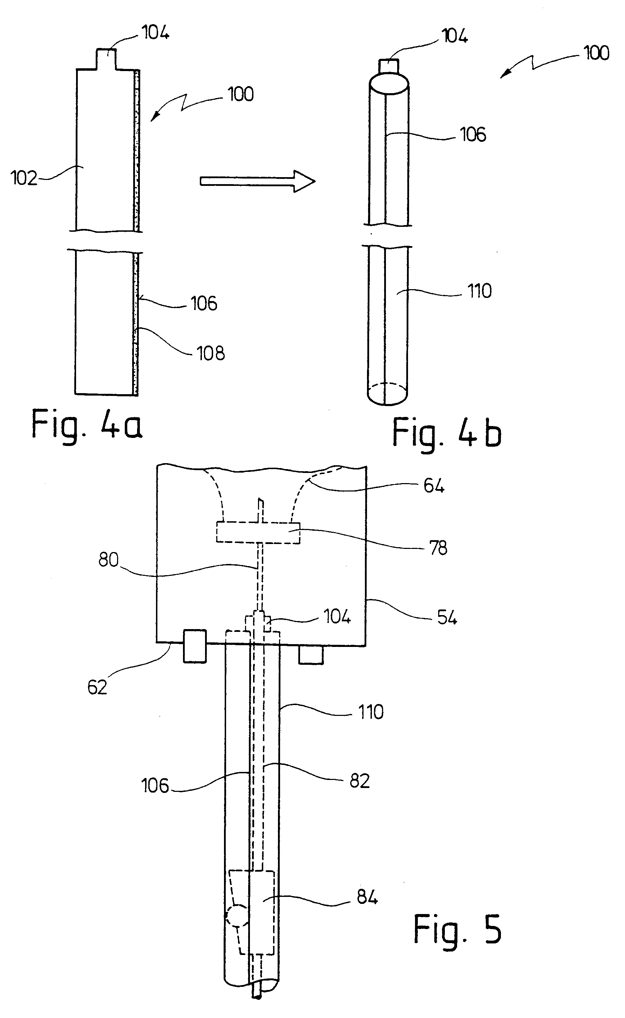

In den Figuren 4a und 4b ist ein Fortsatz 100 dargestellt, der dazu dient, beispielsweise an den in Fig. 3 dargestellten Beutel 54 derart angebracht zu werden, daß der von der Infusionsflasche 64 abführende Schlauch 82 und gegebenenfalls vorhandene Reguliervorrichtungen 84 ebenfalls vor Lichteinwirkung geschützt werden können.

Dazu ist der in Fig. 4a dargestellte Streifen 102 ebenfalls aus lichtundurchlässigem Material hergestellt, beispielsweise aus demselben Material wie der Beutel 54.

Der Streifen 102 ist im Bereich einer Längskante 106 mit einem Haftmittel 108 versehen.

An dem in Fig. 4a oberen Ende ist der Streifen mit einem Ansatzstück 104 versehen, dessen Zweck nachfolgend noch beschrieben wird.

Der Streifen 102 weist dabei eine solche Länge auf, daß er den gesamten Weg eines von der Infusionsflasche 64 abführenden Schlauches 82 bis zur Eindringstelle am Körper des Patienten abdecken kann.

Die Breite des Streifens 102 ist dabei derart, daß er um den Schlauch 82 und auch um eventuell vorhandene Reguliervorrichtungen 84 herumgelegt und zu einem schlauchartigen Gebilde 110 geformt werden kann, wie dies in Fig. 4b dargestellt ist. Dazu wird die mit dem Haftmittel 108 versehene Längskante 106 über die ihr gegenüber liegende Kante gelegt und beispielsweise durch Aufdrücken fest mit dieser verbunden, so daß, wie aus Fig. 4b zu entnehmen, ein schlauchartiges oder rohrförmiges Gebilde 110 entsteht.

Am oberen Ende steht das Ansatzstück 104 vor, das dazu dient, daß das schlauchartige Gebilde 110 über dieses Ansatzstück 104, das ebenfalls mit einem Haftmittel versehen sein kann, an der Innenseite des Beutels 54, wie das in Fig. 5 dargestellt ist, haftend angebracht werden kann.

Es ist selbstverständlich möglich, zunächst den in Fig. 4a dargestellten Streifen 102 auf die notwendige Länge abzulängen und anschließend über das Ansatzstück 104 an der Innenseite des bereits über die Infusionsflasche geschobenen Beutels 54 anzubringen. Anschließend wird der Streifen 102 von Hand um den Schlauch 82 und die Reguliervorrichtung 84 gelegt und zu dem schlauchartigen Gebilde 110 geformt, wie dies in Fig. 5 dargestellt ist.

In weiteren, hier nicht dargestellten Ausführungsbeispielen ist vorgesehen, den Fortsatz 110 bereits bei der Herstellung des Beutels 54 anzubringen, entweder als integralen Bestandteil des Beutels oder durch nachträgliches Verbinden, beispielsweise Verkleben oder Verschweißen mit dem Beutel.

Darüber hinaus ist vorgesehen, schon bei der Herstellung den Fortsatz als fertigen Schlauch auszubilden, der entweder als integraler Bestandteil des Beutels 54 ausgebildet ist, oder der als separates Teil ausgebildet ist, das zunächst über den Schlauch 82 und die Reguliervorrichtung 84 geschoben wird, und erst dann mit dem Beutel verbunden wird.

## Patentansprüche

1. Lichtschutzvorrichtung für den lichtempfindlichen Inhalt einer Infusionsflasche (64), dadurch gekennzeichnet, daß eine lichtundurchlässige Schutzfolie (12, 32) vorgesehen ist, die im Bedarfsfalle nachträglich auf die Infusionsflasche (64) aufbringbar ist, und die derart ausgebildet ist, daß im Bodenbereich der Infusionsflasche (64) Aufhängevorrichtungen (70) für die Infusionsflasche (64) durch die Schutzfolie (12, 32) hindurchbringbar sind, und daß im Auslaufbereich der Infusionsflasche (64) eine Öffnung (22, 42, 62) in der Schutzfolie (12) vorgesehen ist.

2. Lichtschutzvorrichtung nach Anspruch 1, dadurch gekennzeichnet, daß die Schutzfolie (12, 32) schlauchartig ausgebildet ist und auf die Infusionsflasche (64) aufschiebbar ist.

3. Lichtschutzvorrichtung nach Anspruch 1 oder 2, dadurch gekennzeichnet, daß die Schutzfolie (12, 32) als einseitig geschlossener Beutel (14, 34, 54) ausgebildet ist, der über die Infusionsflasche (64) schiebbar ist.

4. Lichtschutzvorrichtung nach Anspruch 3, dadurch gekennzeichnet, daß der Beutel (14, 34, 54) vom Boden (66) der Infusionsflasche (64) her auf diese aufschiebbar ist, so daß bei aufgeschobenem Beutel (14, 34, 54) der Boden (66) der Infusionsflasche (64) in der Nähe des geschlossenen Bodens (16, 36, 56) des Beutels (14, 34, 54) zum Liegen kommt.

5. Lichtschutzvorrichtung nach Anspruch 3 oder 4, dadurch gekennzeichnet, daß im Boden (36, 56) des Beutels (14, 54) eine Öffnung (38, 58) vorgesehen ist, durch die die Aufhängevorrichtung (70) durchführbar ist.

6. Lichtschutzvorrichtung nach Anspruch 5, dadurch gekennzeichnet, daß die Öffnung (38, 58) als Schlitz (38, 58) ausgebildet ist.

7. Lichtschutzvorrichtung nach Anspruch 3 oder 4, dadurch gekennzeichnet, daß im Boden (16) des Beutels (14) eine Perforation (24) eingebracht ist, die zur Schaffung einer Öffnung für den Durchtritt der Aufhängevorrichtung (70) aufreißbar ist.

8. Lichtschutzvorrichtung nach einem der Ansprüche 1 bis 7, dadurch gekennzeichnet, daß an der Schutzfolie Verschlußmittel (38, 44, 46, 94, 94') vorgesehen sind, die nach Anbringen der Schutzfolie an die Infusionsflasche (64) ein Verschleißen von Öffnungen (22, 42, 62; 38, 58) in der Schutzfolie ermöglichen.

9. Lichtschutzvorrichtung nach Anspruch 8, dadurch gekennzeichnet, daß als Verschlußmittel Einschnürmittel (44, 46) vorgesehen sind, die ein Einschnüren der Schutzfolie (32) nach Aufbringen auf die Infusionsflasche (64) ermöglichen.

10. Lichtschutzvorrichtung nach Anspruch 9, dadurch gekennzeichnet, daß die Einschnürmittel zumindest einen umfänglich um die Schutzfolie (32) angeordneten Faden (46) aufweisen.

11. Lichtschutzvorrichtung nach einem der Ansprüche 8 bis 10, dadurch gekennzeichnet, daß als Verschlußmittel Haft- bzw. Klebestreifen oder -laschen (38, 94, 94') vorgesehen sind, die nach Anbringen der Schutzfolie an die Infusionsflasche (64) ein Verschließen von Öffnungen (38, 62) in der Schutzfolie ermöglichen oder ein Verschlossenhalten von gerafften Bereichen der Schutzfolie sicherstellen.

12. Lichtschutzvorrichtung nach einem der Ansprüche 1 bis 11, dadurch gekennzeichnet, daß ein laschenartiger Bereich der Schutzfolie von dieser abhebbar und wieder rücklegbar ist, wodurch bei abgehobenem laschenartigen Bereich eine Öffnung in der Schutzfolie kurzzeitig geschaffen wird, durch die hindurch der Inhalt der durch die Schutzfolie ummantelten Infusionsflasche (64) visuell kontrollierbar ist.

13. Lichtschutzvorrichtung nach einem der Ansprüche 1 bis 12, dadurch gekennzeichnet, daß die Schutzfolie mit einem Fortsatz (100) versehen ist, der eine von der Infusionsflasche (64) abführende Leitung umhüllt, so daß eine von der Infusionsflasche (64) durch die Leitung abgeführte Flüssigkeit vor Lichteinwirkung schützbar ist.

14. Lichtschutzvorrichtung nach Anspruch 13, dadurch gekennzeichnet, daß der Fortsatz (100) als Schlauch ausgebildet ist.

15. Lichtschutzvorrichtung nach Anspruch 13, dadurch gekennzeichnet, daß der Fortsatz (100) als Streifen (102) ausgebildet ist, der durch Umlegen um die Leitung zu einem schlauchartigen Gebilde (110) formbar ist.

16. Lichtschutzvorrichtung nach Anspruch 15, dadurch gekennzeichnet, daß der Streifen (102) längs einer Längskante (106), die längs der zu schützenden Leitung verläuft, mit einem Haftmittel (108) versehen ist.

17. Lichtschutzvorrichtung nach einem der Ansprüche 13 bis 16, dadurch gekennzeichnet, daß der Fortsatz (100) als getrenntes Teil vorgesehen ist, das nachträglich an die Schutzfolie haftend anbringbar ist.

## Claims

1. Light-protection device for protecting the photosensitive content of an infusion flask (64), characterised in that a non-translucent protective foil (12, 32) is provided, which if necessary can be applied to the infusion flask (64) subsequently and which is so formed that in the bottom region of the infusion flask (64) suspending devices (70) for the infusion flask (64) can be passed through the protective foil (12, 32), and in that in the discharge region of the infusion flask (64) an aperture (22, 42, 62) is provided in the protective foil (12).

2. Light-protection device according to claim 1, characterised in that the protective foil (12, 32) has a sheath-like form and can be pushed over the infusion flask (64).

3. Light-protection device according to claim 1 or 2, characterised in that the protective foil (12, 32) is formed as a bag (14, 34, 54) which is sealable at one end and can be pushed over the infusion flask (64).

4. Light-protection device according to claim 3, characterised in that the bag (14, 34, 54) can be pushed over the infusion flask (64) from the bottom (66) of the flask upward, so that when the bag (14, 34, 54) is on, the bottom (66) of the infusion flask (64) ends up in the vicinity of the sealed bottom (16, 36, 56) of the bag (14, 34, 54).

5. Light-protection device according to claim 3 or 4, characterised in that an aperture (38, 58) is provided in the bottom (36, 56) of the bag (14, 54), through which the suspending device (70) can be passed.

6. Light-protection device according to claim 5, characterised in that the aperture (38, 58) is formed as a slot (38, 58).

7. Light-protection device according to claim 3 or 4, characterised in that a perforation (24) is formed in the bottom (16) of the bag (14), which can be torn open to form an aperture for the suspending device (70) to pass through.

8. Light-protection device according to one of claims 1 to 7, characterised in that fastening means (38, 44, 46, 94, 94') are provided on the protective foil to permit closure of the apertures (22, 42, 62; 38, 58) after the protective foil has been applied to the infusion flask (64).

9. Light-protection device according to claim 8, characterised in that laces (44, 46) are provided as fastening means for lacing up the protective foil (32) applied to the infusion flask (64).

10. Light-protection device according to claim 9, characterised in that the lacing-up means have at least one thread (46) wound around the circumference of the protective foil (32).

11. Light-protection device according to one of claims 8 to 10, characterised in that as fastening means adhesive strips or flaps (38, 94, 94') are provided, which after application of the protective foil to the infusion flask (64) permit fastening of apertures (38, 62) in the protective foil or ensure that gathered areas of the protective foil are kept closed.

12. Light-protection device according to one of claims 1 to 11, characterised in that a flap-like region of the protective foil can be removed and put back in place, whereby with the flap-like region removed an aperture is briefly formed in the protective foil through which the content of the infusion flask (64) wrapped in the protective foil can be visually monitored.

13. Light-protection device according to one of claims 1 to 12, characterised in that the protective foil is provided with an extension (100), which envelops a line passing from the infusion flask (64) so that fluid passing through the line from the infusion flask (64) can be protected from the effect of light.

14. Light-protection device according to claim 13, characterised in that the extension (100) is formed as a sheath.

15. Light-protection device according to claim 13, characterised in that the extension (100) is formed as a strip (102) capable of being formed into a sheath-like configuration (110) by wrapping around the line.

16. Light-protection device according to claim 15, characterised in that the strip (102) is provided with an adhesive (108) along one longitudinal edge (106) extending along the line to be protected.

17. Light-protection device according to one of claims 13 to 16, characterised in that the extension (100) is a separate part which can be assembled subsequently by adhesion to the protective foil.

## Revendications

1. Dispositif de protection contre la lumière pour le contenu sensible à la lumière d'une bouteille de perfusion (64), caractérisé en ce qu'il est prévu un film protecteur (12, 32) opaque, qui peut être appliqué ensuite, si besoin est, sur la bouteille de perfusion (64) et qui est constitué de telle sorte que des dispositifs de suspension (70) pour la bouteille de perfusion (64) peuvent être passés à travers le film protecteur (12, 32) au niveau du fond de la bouteille de perfusion (64) et en ce qu'au niveau du goulot de la bouteille de perfusion (64), une ouverture (22, 42, 62) est prévue dans le film protecteur (12).

2. Dispositif de protection contre la lumière selon la revendication 1, caractérisé en ce que le film protecteur (12, 32) se présente sous la forme d'un tube pouvant être enfilé sur la bouteille de perfusion (64).

3. Dispositif de protection contre la lumière selon les revendications 1 ou 2, caractérisé en ce que le film protecteur (12, 32) se présente sous la forme d'une poche (14, 34, 54) fermée d'un côté, pouvant être glissée sur la bouteille de perfusion (64).

4. Dispositif de protection contre la lumière selon la revendication 3, caractérisé en ce que la poche (14, 34, 54) peut être enfilée par le fond (66) de la bouteille de perfusion (64), de telle sorte que lorsque la poche (14, 34, 54) est en place, le fond (66) de la bouteille de perfusion (64) vient se loger au niveau du fond fermé (16, 36, 56) de la poche (14, 34, 54).

5. Dispositif de protection contre la lumière selon les revendications 3 ou 4, caractérisé en ce que, dans le fond (36, 56) de la poche (14, 54), il est prévu une ouverture (38, 58) par laquelle peut être passé le dispositif de suspension (70).

6. Dispositif de protection contre la lumière selon la revendication 5, caractérisé en ce que l'ouverture (38, 58) est en forme de fente (38, 58).

7. Dispositif de protection contre la lumière selon les revendications 3 ou 4, caractérisé en ce que, dans le fond (16) de la poche (14), il est prévu une perforation (24) qui peut être déchirée pour créer une ouverture pour le passage du dispositif de suspension (70).

8. Dispositif de protection contre la lumière selon l'une quelconque des revendications 1 à 7, caractérisé en ce que le film protecteur comporte des moyens de ficelage (38, 44, 46, 94, 94') permettant de refermer les ouvertures (22, 42, 62 ; 38, 58) ménagées dans le film protecteur, après la mise en place du film protecteur sur la bouteille de perfusion (64).

9. Dispositif de protection contre la lumière selon la revendication 8, caractérisé en ce qu'il est prévu, en tant que moyen de fermeture, un moyen de ficelage (44, 46) permettant de ficeler le film protecteur (32) après sa mise en place sur la bouteille de perfusion (64).

10. Dispositif de protection contre la lumière selon la revendication 9, caractérisé en ce que le moyen de ficelage comporte au moins un fil (46) disposé sur le pourtour du film protecteur (32).

11. Dispositif de protection contre la lumière selon l'une quelconque des revendications 8 à 10, caractérisé en ce que le moyen de fermeture peut être constitué par des bandes ou languettes (38, 94, 94') collantes ou adhésives qui assurent, après mise en place du film protecteur sur la bouteille de perfusion (64), une fermeture des ouvertures (38, 62) du film protecteur ou maintiennent fermées les zones resserrées du film protecteur.

12. Dispositif de protection contre la lumière selon l'une quelconque des revendications 1 à 11, caractérisé en ce qu'une partie en forme de languette du film protecteur peut être soulevée et remise en place, le soulèvement de cette partie en forme de languette permettant de réaliser temporairement une ouverture dans le film protecteur, à travers laquelle il est possible de contrôler visuellement le contenu de la bouteille de perfusion (64) enveloppée dans le film protecteur.

13. Dispositif de protection contre la lumière selon l'une quelconque des revendications 1 à 12, caractérisé en ce que le film protecteur comporte un prolongement (100) qui entoure un conduit sortant de la bouteille de perfusion (64), de telle sorte qu'un liquide sortant de la bouteille de perfusion (64) par ce conduit puisse être protégé de l'action de la lumière.

14. Dispositif de protection contre la lumière selon la revendication 13, caractérisé en ce que le prolongement (100) se présente sous la forme d'un tube.

15. Dispositif de protection contre la lumière selon la revendication 13, caractérisé en ce que le prolongement (100) se présente sous la forme d'une bande (102) pouvant être disposée autour du conduit pour former un élément (110) tubulaire.

16. Dispositif de protection contre la lumière selon la revendication 15, caractérisé en ce qu'un bord longitudinal (106) de la bande (102), disposé le long du conduit à protéger, comporte un adhésif (108).

17. Dispositif de protection contre la lumière selon l'une quelconque des revendications 13 à 16, caractérisé en ce que le prolongement (100) est prévu en tant qu'élément séparé pouvant être fixé ensuite par collage sur le film protecteur.
